Europäisches Patentamt

European Patent Office

Office européen des brevets

(19)

(11) Veröffentlichungsnummer: **0 243 530 B1**

(12) **EUROPÄISCHE PATENTSCHRIFT**

(45) Veröffentlichungstag der Patentschrift: **29.05.91**

(51) Int. Cl.⁵: **G01N 1/28**, G01N 31/22

(21) Anmeldenummer: **86109820.0**

(22) Anmeldetag: **17.07.86**

(54) Verfahren zur Feststellung von noch lebenden Hausstaubmilben.

(30) Priorität: **19.07.85 DE 3525883**

(43) Veröffentlichungstag der Anmeldung:
**04.11.87 Patentblatt 87/45**

(45) Bekanntmachung des Hinweises auf die
Patenterteilung:
**29.05.91 Patentblatt 91/22**

(84) Benannte Vertragsstaaten:
**AT BE CH DE FR GB IT LI LU NL SE**

(56) Entgegenhaltungen:
**EP-A- 0 144 820**
**DE-A- 3 404 821**

(73) Patentinhaber: **Gesellschaft für hausbiologische Forschung mbH
Rheinallee 96
W-6500 Mainz 1(DE)**

(72) Erfinder: **Bischoff, Edelbert, Dr.
Leibnizstrasse 52
W-6719 Kirchheim-Bolanden(DE)**
Erfinder: **Wetter, Gert
Hauptstrasse 8
W-6501 Wörrstadt 2(DE)**

(74) Vertreter: **Zumstein, Fritz, Dr. et al
Dr. F. Zumstein Dr. E. Assmann Dipl.-Ing. F.
Klingseisen Bräuhausstrasse 4
W-8000 München 2(DE)**

**Beschreibung**

In zahlreichen Ländern stellen Kleinschädlinge wie Hausstaubmilben in zunehmendem Maß eine Belästigung dar, von der insbesondere Allergiker betroffen sind. Hausstaubmilben vermehren sich in geeigneten Biotopen sehr stark und erzeugen während ihrer Lebensperiode eine Menge allergenhaltiger Exkremente, die ihr Körpergewicht um ein Vieltausendfaches übertrifft. Exkremente, tote und lebende Milben befinden sich dann im Hausstaub, der besonders in textilen Gebilden innerhalb der Häuser wie Matratzen, Polstermöbeln und Teppichen anzutreffen ist und als Feinststaub beim Benutzen bzw. Begehen der textilen Gegenstände aufgewirbelt wird. Hausstaub-Allergien können sich bis zu lebensbedrohenden Beschwerden steigern.

Will man Hausstaubmilben als Quelle weiteren allergenhaltigen Staubes beseitigen, so stellen sich folgende Fragen: Wo halten sich die Milben auf? - In welcher Menge sind lebende Milben vorhanden? - Welche Milbensorten kommen in welchen Entwicklungszuständen vor? - Hat die Anwendung mechanischer Mittel, wie Saugen oder Klopfen eine Wirkung erbracht? - Konnte nach Anwendung eines akariziden Mittels die Milben-Population reduziert oder beseitigt werden? - Hat das Mittel eine remanente Wirkung? - Können sich aus den noch vorhandenen Eiern neue Milben entwickeln? - Nach welchem Zeitraum findet eine Neubesiedlung durch Hausstaubmilben statt? - usw.

Zur Beantwortung derartiger Fragen ist man derzeit darauf angewiesen, den durch Staubsaugen gewonnenen Staub auf die Anwesenheit von Milben hin zu untersuchen. Das bewährteste Verfahren hierfür ist das Flotationsverfahren. Dabei wird der Staub in ein geeignetes, flüssiges Medium überführt, in dem tote und lebende Milben aufschwimmen. Von da werden die Milben mit Hilfe einer Präpariernadel auf einen Objektträger übertragen und dann mikroskopisch identifiziert. Die Unterscheidung zwischen toten und lebenden Milben bedarf dabei großer Erfahrung. Sie stützt sich auf die Beobachtung, daß lebendige Milben in der Regel frei von Beschädigungen sind. Tote Milben aber, bzw. solche, die bereits vor Entnahme der Staubprobe tot waren, haben immer irgendwelche Glieder, Kauwerkzeuge oder Stacheln verloren. Als lebend werden Milben angesprochen, die noch erkennbar lebendig oder unbeschädigt und prall gefüllt sind. Ein Teil der Milben überlebt nämlich die Identifizierungs-Vorgänge nicht.

Der wesentliche Nachteil dieser Methode liegt aber nicht an Schwierigkeiten der Identifikation lebendiger Milben. Er ist vielmehr verfahrensbedingt. In einer Staubprobe können nämlich immer nur die Hausstaubmilben nachgewiesen werden, die beim Absaugen in die Staubprobe gelangt sind. Es gibt aber keine Auskunft darüber, welcher Anteil an Staub bzw. Milben überhaupt durch Absaugen aus einem textilen Gebilde erfaßt werden kann.

Neuere Untersuchungen haben gezeigt, daß beim ein- oder zweimaligen Absaugen z.B. eines Teppichs nur ein beschränkter Anteil des vorhandenen Staubes einschließlich toter und lebender Milben erfaßt wird.

Außerdem führt die Untersuchung einer Staubprobe immer nur zu einer Aussage über das gesamte Flächengebilde, von dem diese Probe entnommen wurde.Eine Angabe darüber, wo sich die Milben, insbesondere die, lebenden, tatsächlich befinden, erhält man auf diesem Weg nicht.,

Dies gilt ebenfalls für die in EP-A-O 144 820 beschriebene Untersuchung auf etwa allergenhaltigen Hausstaub; denn auch dort erfolgt die Probenentnahme des zu untersuchenden Staubes mittels eines Staubsaugers.

Damit stellte sich die Aufgabe, ein Verfahren zu entwickeln, mit dessen Hilfe unter Labor- und Praxisbedingungen zuverlässige und schnelle Aussagen über die Anwesenheit lebender Hausstaubmilben zu gewinnen wären. Von Interesse war ferner, eine Auskunft über die Verteilung der Milben über das untersuchte Flächenelement zu erhalten, sowie über eine einfache Möglichkeit zu verfügen, die lebenden Milben hinsichtlich ihrer Art, ihres Geschlechtes und ihres Entwicklungszustandes mikroskopisch zu beurteilen. Diese Aufgabe wird mit dem erfindungsgemäßen Verfahren gelöst.

Das Verfahren berücksichtigt die ökologischen Bedingungen eines Milben-Biotops sowie die Mobilität der Milben. Obgleich Hausstaubmilben für sie ungünstige ökologische Bedingungen, z.B. tiefere Temperaturen, längere Zeit ertragen können und besonders ihre Eier relativ unempfindlich gegen Klimaschwankungen sind, benötigen Populationen, die sich entwickeln sollen, ein Kleinklima von ca. 25°C und 65-85 % relativer Luftfeuchtigkeit.

Verändert man nun diese Bedingungen, so versuchen die Milben ein Flächenelement zu erreichen, das ihnen günstigere Existenzmöglichkeiten bietet. So weichen sie erhöhter Temperatur (Wärme) und damit verbundener veränderter relativer Luftfeuchtigkeit aus. Bei einer bevorzugten Ausführungsform des erfindungsgemäßen Verfahrens wird dies zur Herbeiführung einer erzwungenen, gezielten Wanderung benutzt.

Eine einfachere Ausführungsform des erfindungsgemäßen Verfahrens für Fälle, bei denen auf die Einwirkung von Wärme verzichtet werden muß, macht lediglich von der natürlichen Mobilität der Milben Gebrauch.

Gegenstand der vorliegenden Erfindung ist deshalb ein Verfahren zur Feststellung von noch lebenden Hausstaubmilben gemäß den Patentansprüchen.

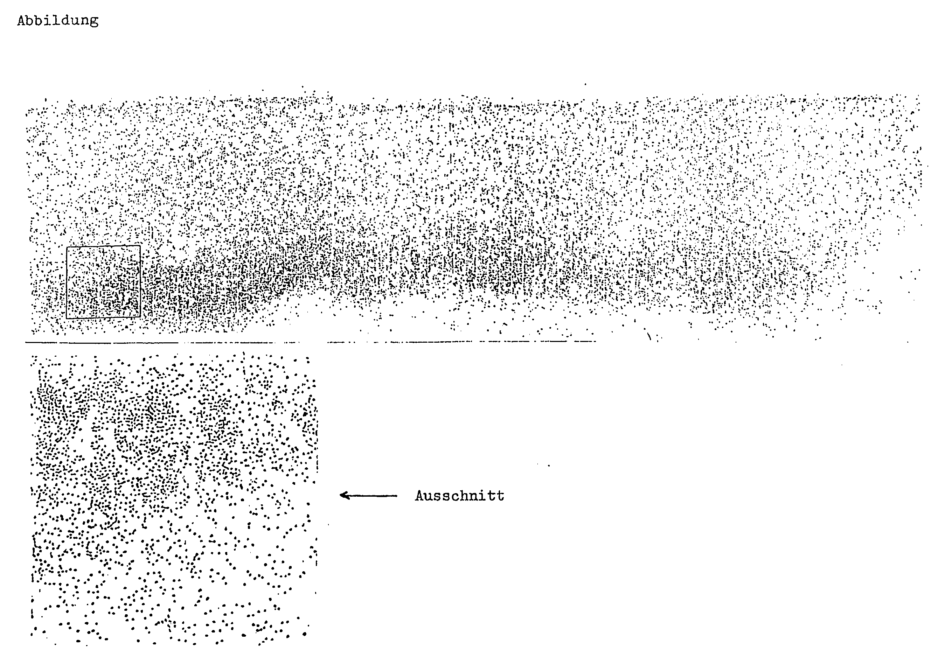

Die Abbildung zeigt die Verteilung von Kleinschädlingen über ein textiles Flächenelement von 0,25m², ethalten nach dem Wärmefluchtverfahren (Beispiel B, Tab.2, Versuch 1).

Die nachstehenden Ausführungen dienen der näheren Beschreibung und Erläuterung dieses erfindungsgemäßen Verfahrens.

Der auf Milbenbefall zu prüfende Gegenstand ist ein textiler, flächenförmiger Gegenstand. Als solche textilen Flächengebilde sind beispielsweise Teppiche, Polstermöbel, Matratzen, Betten, textile Wandverkleidungen etc. zu nennen.

Als Haftklebefolie sind die üblichen bekannten Produkte geeignet. Besonders bevorzugt sind solche Haftklebefolien, deren Kleber zwar die Milben (anhaftend) aufnimmt, jedoch nicht auf den zu prüfenden Gegenstand transferiert wird.

Um eine verbesserte Auflage der Haftklebefolie mit der Vorderseite bzw. Oberfläche des textilen Gegenstandes herzustellen, wird es bevorzugt, die Haftklebefolie ihrerseits mit einem geeigneten plattenförmigen Gegenstand (wie z.B. einer Glasplatte) zu beschweren. Wegen der Lichtempfindlichkeit von Hausstaubmilben wird es ferner bevorzugt, wenn die Beschwerung mittels einer abgedunkelten bzw. lichtundurchlässigen Platte oder mittels einer (z.B. mit einer lichtundurchlässigen Folie oder Platte) beschichteten Glasplatte erfolgt.

Zur erleichterten Handhabung der mit Milben, beladenen Haftklebefolie, wird diese bevorzugt auf ihrer Klebeseite mit einer durchsichtigen, nichtklebenden Schutzfolie beschichtet. Als derartige Schutzfolie können die üblichen Kunststoff-Folien (wie z.B. Polyäthylenfolien) verwendet werden.

In einer weiteren bevorzugten Ausführungsform können der Klebeschicht der Haftklebefolie auch noch Substanzen beigefügt sein, die die zu bewertenden Milben anlocken oder anfärben. Beispiele für solche Lockstoffe sind Pheromone oder dergleichen. Ein Beispiel für das Anfärben gemäß den Patentansprüchen 6 und 7 ist die kombinierte Extraktion und Azofarbstoffbildung mit dem Exkrementebestandteil Guanin, wie in der EP-A-O 144 820 beschrieben; auch die Schutzfolie gemäß Patentanspruch 4 kann eine solche Azokupplungskomponente oder Extraktionsmittel enthalten.

Bei der einfachsten Ausführungsform des erfindungsgemäßen Verfahrens werden die Milben aufgrund ihrer natürlichen Mobilität (Wanderung) an der Vorderseite bzw. Oberfläche des Gegenstandes aufgenommen, also ohne Anwendung einer Wärmequelle. Diese Methode ist insbesondere bei solchen Gegenständen angebracht, die man (von ihrer Rückseite her) keiner Wärmeeinwirkung unterwerfen kann. Bereits in diesem Falle liefert das erfindungsgemäße Verfahren eine Aussage über das Vorhandensein lebender Milben.

Auch lassen sich bereits in diesem Fall noch quantitative Bewertungen durchführen, wie die nachstehenden Ausführungen (vgl. unter II) zeigen.

Bei einer anderen Ausführungsform des erfindungsgemäßen Verfahrens arbeitet man bevorzugt in der Weise, daß man den zu prüfenden Gegenstand auf seiner Rückseite der Einwirkung von Wärme unterwirft und die Milben zu einer gesteigerten Wanderung an die Vorderseite bzw. Obefläche veranlaßt. Bei dieser Ausführungsform erfolgt die Einwirkung von Wärme bevorzugt mittels einer flächenförmigen Wärmequelle. Dies kann beispielsweise eine beheizbare Fläche (Wärmebank) aus Metall, evtl. auch aus Keramik, Glas oder geeignetem Kunststoff sein. Ferner kann die flächenförmige Wärmequelle auch in der Form eines gegebenenfalls modifizierten Heizkissens eingesetzt werden. Diese Ausführungsform des erfindungsgemäßen Verfahrens (mit Wärmeeinwirkung) wird ebenfalls nachstehend (unter I) erläutert.

Es können aber auch die beiden vorstehend genannten Ausführungsformen (nämlich II, aufgrund der natürlichen Mobilität; und I, aufgrund von Wärmeeinwirkung) miteinander kombiniert werden. Die Ergebnisse einer derartigen Arbeitsweise sind nachstehend (unter III) erläutert.

I . Methode mit Wärmeeinwirkung

Ein (auf Hausstaubmilben) zu prüfendes textiles Flächengebilde wird auf eine sogenannte Wärmebank gelegt. Dies ist bevorzugt eine Metallfläche, die in irgendeiner Form gleichmäßig beheizt werden kann. Als Beispiel sei ein Aluminiumblock der Größe 120 X 30 cm und einer Dicke von 3 cm genannt. Innerhalb des Blockes liegt eine Kupferrohrleitung in parallel geführten Windungen, die in Kristallquarzsand eingebettet ist. die Rohrleitung ist mit einem Thermostat verbunden, der Wärmeträgeröl der gewünschten Temperatur durch das System leitet.

Legt man ein Teppichstück mit seiner Rückseite auf eine solche Wärmebank, so erwärmt sich nach Einschalten der Heizung, von Zimmertemperatur ausgehend, das Wärmeträgeröl bis auf 150° C. Der Teppichflor erreicht dabei nach ca. 60 Min. eine Temperatur von ca. 70° C.

Während dieser Zeit (60 Min.) verlassen die Milben sukzessive ihr Biotop in der Tiefe des Teppichflors und wandern in Richtung Teppichoberfläche.

Zu Beginn des Versuchs legt man auf die Teppichoberfläche eine Haftklebefolie, die die ankommenden Milben aufnimmt und deren Klebeschicht vorzugsweise in ihrer Konsistenz so gewählt ist, daß der Teppich nicht mit Kleber beschmutzt wird. Um einen gleichmäßigen Kontakt zwischen Klebefolie und Teppichoberfläche herzustellen, wird vorzugsweise die Klebefolie ihrerseits mit einer geeigneten Platte (z.B. Glasplatte) beschwert. Um der Lichtempfindlichkeit der Hausstaubmilben Rechnung zu tragen, wird vorzugsweise eine dunkle Platte verwendet oder die Glasplatte mit einer dunklen bzw. lichtundurchlässigen Folie oder Platte abgedeckt.

Die Klebefolie wird dann vom Teppichflor abgezogen und vorzugsweise zur Fixierung des Ergebnisses mit einer Polyäthylenfolie (Schutzfolie) auf der Klebeseite überschichtet. An diesem Gebilde kann dann die Auszählung, Identifizierung und Lokalisierung der Hausstaubmilben vorgenommen werden. Auch die Abdeckfolie kann einen Reaktionspartner für ein Milbenerkennungsverfahren, z .B. eine Anfärbereaktion erhalten, bei einer beabsichtigen Azofarbstoffbildung z.B. eine Azokupplungskomponente oder ein Extraktionsmittel.

Auf das untersuchte Teppichstück kann sofort eine zweite Klebefolie in der beschriebenen Weise gelegt werden, die dort unter den gleichen Bedingungen eine weitere Stunde lang verbleibt. Dabei steigert man die Temperatur in der Tiefe des Flors auf ca. 100° C. Auf diese Weise wird festgestellt, ob noch weitere Milben die Klebefolie erreichen. In der Regel erhält man dabei jedoch nur noch einige Prozent der ursprünglich festgestellten Anzahl von Milben.

(Als Klebefolie hat sich das Handelsprodukt der Fa. Beiersdorf mit der Bezeichnung "Tesa Schutzfolie 5609" bewährt.)

Setzt man abgezählte Milben auf textilen Oberflächen aus und läßt sie sich eine bestimmte Zeit verkriechen, so findet man bei Durchführung des erfindungsgemäßen Verfahrens die Hauptmenge (ca. 2/3) der eingesetzten Milben an der Klebefolie (Beispiel A). Sie sind dort in der Regel noch erkennbar lebendig und lassen sich mikroskopisch hinsichtlich Milbensorte, -geschlecht und -entwicklungszustand identifizieren.

Mit Hilfe dieses Verfahrens können aber auch natürlich milbenbesiedelte Teppiche und andere textile Flächengebilde untersucht und weitgehend quantitativ bewertet werden. Das gleiche gilt für Teppichstücke und andere Textilien, die als Proben aus Haushalten von Allergikern entnommen wurden.

Das Verfahren läßt sich ferner auch an Ort und Stelle, d.h. in Haushalten von Allergikern durchführen, indem eine mobile flächenförmige Wärmequelle, z.B. in Form eines gegebenenfalls modifizierten Heizkissens unter die Teppiche geschoben wird, die nicht vollflächig verklebt sind. Entsprechenderweise können auch Polstermöbel untersucht werden, vorzugsweise wenn diese - wie bei neueren Möbeln häufig anzutreffen - durch einen Reißverschluß zu öffnen oder leicht aufzutrennen sind.

Bei Gegenständen, bei denen es nicht möglich ist, eine gerichtete Wärmewirkung zu bewerkstelligen, kann man sich des Auflegens einer vorzugsweise - wie beschrieben - abgedeckten Haft-Klebefolie bedienen, um zunächst eine Aussage darüber zu erhalten, ob mit lebenden Milben zu rechnen ist. Auch mit dieser Methode können noch weitgehend quantitative Bestimmungen durchgeführt werden, wenn auch mit verringerter Zuverlässigkeit (siehe Abschnitt II).


Beispiel A: Wiedergewinnung eingesetzter Kleinschädlinge

Zur Bewertung des "Wärmeflucht"-Verfahrens hinsichtlich Aussagekraft und Ausbeute werden Teppichstücke (Polyamid-Velours-Rohweiß-Ware) der Größe 0,25 m² in jeweils drei gleiche Teile geteilt. Auf jedes dieser Teile werden zwischen 40 und 200 Milben ausgesetzt. Nach einer Verkriechzeit von 2 Stunden legt man eine Haftklebefolie auf die Teppichstücke, deckt sie mit einer Platte ab und beginnt mit der Wärmeeinwirkung, wie oben unter I. beschrieben. Das Ergebnis wird - wie ebenfalls oben beschrieben - abschließend nach 1 und 2 Stunden beurteilt.


Ergebnis (Beispiel entsprechend Versuch Nr. 3 in Tab. 1 )

1. Drittel: ausgesetzt 75 Milben, Folienausbeute 51 Milben
2. Drittel: ausgesetzt 171 Milben, Folienausbeute 101 Milben
3. Drittel: ausgesetzt 122 Milben, Folienausbeute 82 Milben.

4

Zusammenfassung:

Ausgesetzt 368 Milben, Folienausbeute 234 Milben
Anteil gefundener Milben: 64 %.

Wiederholungen dieses Versuches (vgl. die Nummern 1, 2, 4 und 5 mit jeweils 3 Teilstücken in Tab. 1 ) führen zu Ergebnissen mit der gleichen Größenordnung für die Milbenausbeute.

Der Anteil gefundener Milben liegt im Mittel bei 65 % + 5 % (daher wird nachstehend der Faktor 100 : 65 verwendet). Einzelheiten sind Tab. 1 zu entnehmen.

Beispiel B: Wärmefluchtverfahren bei natürlich besiedelten Teppichstücken

Nach der Bestimmung des Ausbeutefaktors gemäß Beispiel A kann mit Hilfe des "Wärmeflucht"-Verfahrens die Populationsdichte natürlich besiedelter Teppiche bestimmt, sowie ein Bild der Verteilung über das betreffende Flächenelement erhalten werden (siehe Abbildung).

Bei günstigen Klima- und Futterbedingungen erhöhen sich die Populationen innerhalb einiger Monate von 1000 auf 10 000 bis 50 000 Stück pro 0,25 m²

Einzelheiten sind Tabelle 2 zu entnehmen.

Das erfindungsgemäße Verfahren ermöglicht Aussagen über die Populationsdichte von Milben in textilen Flächengebilden, die dort in einer Dichte bis zu 200 000 Stück pro m² auftreten können; in Extremfällen wurden auch schon 500 000 Stück pro m² beobachtet.


II. Methode ohne Wärmeeinwirkung aufgrund der natürlichen
   Mobilität


Dabei wird eine Haft-Klebefolie auf die zu untersuchende textile Fläche, wie Teppich, Matratze oder Polstermöbel gelegt, vorzugsweise abgedeckt, nach einer Verweilzeit von bis zu 24 Stunden entnommen (vorzugsweise mit einer Schutzfolie überschichtet) und anschließend ausgewertet.

Nach Aufbringen einer abgezählten Milbenmenge zu Testzwecken und einer Verkriechzeit von 2 Stunden werden bei für Milbenbiotope günstigen Klimabedingungen (25° C, 75 % relative Luftfeuchtigkeit) ca. 30 % der eingesetzten Milben nach einer Auflagezeit von 24 Stunden auf der Folie gefunden. Näheres ist Tab.3 zu entnehmen.

Liegen für die Mobilitätsbewertung der Milben weniger günstige Versuchsbedingungen, z. B. Zimmertemperatur, aber nur geringe Luftfeuchtigkeit vor (25 % relative Luftfeuchtigkeit), so werden immerhin noch ca. 8 % der lebenden Milben festgestellt (siehe Tab. 4).

Beide Fälle sind in der Praxis möglich. Die Milbenausbeute nach dem erfindungsgemäßen Verfahren unter Ausnutzung der eigenen Mobilität der Kleinschädlinge liegt demnach zwischen ca. 8 % und ca. 30 % in Abhängigkeit von den jeweils in der Praxis gegebenen Klimabedingungen.

Bezüglich weiterer Einzelheiten dieser Methode, z.B. der Auswertung der Folien, wird auf die vorstehenden Ausführungen unter I. verwiesen.


III. Kombinierte Anwendung der beiden vorstehenden Methoden
   (Mobilität bei Zimmertemperatur und gerichtete Bewegung
   unter dem Einfluß von Wärme)
   (Auf diesem Wege ist eine ungefähre Ausbeutebewertung des
   Mobilitätstests bei natürlicher Besiedlung textiler
   Flächengebilde möglich. Die kombinierte Methode wurde
   deshalb auch bereits bei den in Tab. 4 geschilderten
   Versuchen angewandt.)


Bei der Kombination der beiden Methoden erhält man zunächst aufgrund der Eigenmobilität der Milben bei günstigen Klimabedingungen eine Milbenausbeute von ca. 30 %. Dies entspricht den bereits in Tab. 3 gefundenen Werten.

Die noch verbleibende Population wird dann der Einwirkung von Wärme, wie unter I. beschrieben, ausgesetzt. Die dabei auf der Klebefolie festgestellte Milbenzahl wird - wie ebenfalls unter I. beschrieben - mit dem Faktor 100:65 hochgerechnet. Die Summe aus dem so erhaltenen Wert nach dem Wärmefluchtverfahren und demjenigen nach dem Mobilitätstest ergibt die Gesamtpopulation und damit auch die Möglichkeit der Bewertung der nach dem Mobilitätstest bestimmten Milbenzahl als prozentualer Anteil dieser Gesamtpopulation.

Näheres ist Tabelle 5 zu entnehmen.

Bezüglich weiterer Einzelheiten wird auf die vorstehenden Ausführungen unter I. und II. verwiesen.

Wie weiter oben in Verbindung mit Ziffer I. ausgeführt, läßt sich durch das erfindungsgemäße Verfahren in der Ausführungsform des "Wärmefluchtverfahrens" die Hauptmenge der vorhandenen lebenden Hausstaubmilben aus dem befallenen Gegenstand austreiben und kann dann (z.B. mittels einer Haftklebefolie) aufgenommen werden. Gegenstand der vorliegenden Anmeldung ist deshalb auch die Anwendung des erfindungsgemäßen Verfahrens für die Bekämpfung (Entfernung) noch lebender Hausstaubmilben wie in Anspruch 11 angegeben. Diese Arbeitsweise ist dadurch gekennzeichnet, daß man den (gegen lebenden Milbenbefall) zu bekämpfenden Gegenstand auf seiner Rückseite der Einwirkung von Wärme unterwirft, dabei die Milben zu einer gesteigerten Wanderung an die Vorderseite bzw. Oberfläche veranlaßt und die ausgewanderten Milben dann aufnimmt sowie gegebenenfalls identifiziert. Auch dabei erfolgt die Wärmeeinwirkung bevorzugt gemäß Patentanspruch 10, d.h. mittels einer flächenförmigen Wärmequelle.

Weitere bevorzugte Ausführungsformen ergeben sich aus den Patentansprüchen 2, 3 und 5. Bezüglich weiterer Einzelheiten für ein solches Bekämpfungsverfahren wird auf die vorstehenden, im Zusammenhang mit dem "Wärmefluchtverfahren" gemachten Ausführungen verwiesen, deren Inhalt hier ebenfalls relevant ist (vgl. auch die entsprechenden Ausführungen unter I., wobei man statt einer zweistufigen Wärmebehandlung natürlich auch eine Wärmebehandlung in einer Stufe durchführen kann).

Das neue Bekämpfungsverfahren kann sowohl mit anschließender Identifizierung der Milben (wie oben erläutert), als auch ohne eine solche Identifizierung durchgeführt werden; im letzteren Fall werden dann die aufgenommenen Milben, z.B. in Form einer beladenen Haftklebefolie, einfach entfernt.

Tabelle 1

Ergebnis des Wärmefluchtverfahrens im Modellversuch auf jeweils 0,25 m$^2$ Teppichfläche
(A: ausgesetzte Milben, B: wiedergefundene Milben)

| Versuch Nr. | 1 | | 2 | | 3 | | 4 | | 5 | |
|---|---|---|---|---|---|---|---|---|---|---|
| | A | B | A | B | A | B | A | B | A | B |
| Teilstück a | 104 | 72 | 57 | 44 | 75 | 51 | 72 | 45 | 115 | 90 |
| Teilstück b | 156 | 102 | 178 | 108 | 171 | 101 | 128 | 83 | 174 | 126 |
| Teilstück c | 47 | 34 | 110 | 58 | 122 | 82 | 161 | 94 | 47 | 20 |
| | 307 | 208 | 345 | 210 | 368 | 234 | 361 | 222 | 336 | 236 |
| Anteil gefundener Milben | | 68 % | | 61 % | | 64 % | | 61 % | | 70 % |

EP 0 243 530 B1

Tabelle 2

Milbenausbeute beim Wärmefluchtverfahren (Temperaturänderung von Zimmertemperatur bis ca. 100°C)
(aus natürlich besiedelten Teppichstücken der Größe 0,25 m²)

|                                                                      | Versuch 1 | Versuch 2 |
|----------------------------------------------------------------------|-----------|-----------|
| ursprüngliche Milbenzahl                                             | 1000      | 1000      |
| Entwicklungszeit in Monaten                                          | 4         | 4         |

Milbenbestimmung nach dem Wärmefluchtverfahren

|                                                                      | Versuch 1 | Versuch 2 |
|----------------------------------------------------------------------|-----------|-----------|
| Gesamtzahl gefundener Milben                                         | 31.050    | 11.930    |
| Gesamtzahl vorhandener Milben (Hochrechnung mit Faktor 100:65)       | 47.770    | 18.350    |

EP 0 243 530 B1

Ergebnis des Mobilitätstests im Modellversuch auf jeweils 0,25 m² Teppichfläche
(A: ausgesetzte Milben, B: wiedergefundene Milben)
(25°C, 75 % relative Luftfeuchtigkeit)

| Versuch Nr. | 1 | | 2 | |
|---|---|---|---|---|
| | A | B | A | B |
| Teilstück a | 70 | 25 | 239 | 69 |
| Teilstück b | 140 | 45 | 180 | 67 |
| Teilstück c | 269 | 69 | 110 | 34 |
| | ___ | ___ | ___ | ___ |
| | 479 | 139 | 529 | 170 |

Anteil gefundener Milben
bei hoher relativer Luftfeuchtigkeit

29 %                                    32 %

EP 0 243 530 B1

Tabelle 4

Milbenausbeute beim Mobilitätstest und niedriger relativer Luftfeuchtigkeit (23°C, 25 % relative Luftfeuchtigkeit)
(aus natürlich besiedelten Teppichstücken der Größe 0,12 m²)

| Versuch Nr. | 1 | 2 | 3 | 4 |
|---|---|---|---|---|
| | Anzahl Milben | Anzahl Milben | Anzahl Milben | Anzahl Milben |
| 1. Mobilitätstest | 971 | 1.125 | 1.548 | 901 |
| 2. Bestimmung der verbliebenen Milben nach dem Wärmefluchtverfahren und Hochrechnung mit dem Faktor 100:65 | 11.145 | 13.255 | 14.271 | 12.954 |
| Gesamtpopulation | 12.116 | 14.380 | 15.819 | 13.855 |
| Milbenausbeute beim Mobilitätstest und niedriger relativer Luftfeuchtigkeit | 8,0 % | 7,8 % | 9,8 % | 6,5 % |

EP 0 243 530 B1

Milbenausbeute beim Mobilitätstest und günstiger relativer Luftfeuchtigkeit (25°C, 75 % relative Luftfeuchtigkeit) und anschließendem Wärmefluchtverfahren (aus natürlich besiedeltem Teppich der Größe 0,25 m² und Untersuchung einzelner Teilstücke der Größe ca. 0,04 m²)

| Versuch Nr. | 1 | 2 | 3 | 4 |
|---|---|---|---|---|
| | Anzahl Milben | Anzahl Milben | Anzahl Milben | Anzahl Milben |
| 1. Mobilitätstest | 3.800 | 3.200 | 3.200 | 2.400 |
| 2. anschließendes Wärmeflucht-verfahren (Direktbestimmung) | 5.650 | 6.000 | 5.000 | 2.650 |
| 3. Hochrechnung der nach 2. erhaltenen Werte mit dem Faktor 100:65 | 8.690 | 9.230 | 7.690 | 4.080 |
| Gesamtpopulation (1 + 3) | 12.490 | 12.430 | 10.890 | 6.480 |
| Milbenausbeute beim Mobilitäts-test und hoher relativer Luft-feuchtigkeit | 30 % | 26 % | 29 % | 37 % |

**Ansprüche**

1. Verfahren zur Feststellung von noch lebenden Hausstaubmilben in einem textilen, flächenförmigen Gegenstand, dadurch gekennzeichnet, daß man bei dem (auf lebenden Milbenbefall) zu prüfenden Gegenstand die Milben an die Vorderseite bzw. Oberfläche des Gegenstandes wandern läßt, die ausgewanderten Milben dort mittels einer Haftklebefolie aufnimmt und sie dann gegebenenfalls abzählt, hinsichtlich ihrer Verteilung bewertet und mikroskopisch identifiziert.

2. Verfahren nach Anspruch 1, dadurch gekennzeichnet, daß man eine Haftklebefolie einsetzt, die zwar die Milben aufnimmt, jedoch keinen Kleber an den zu prüfenden Gegenstand abgibt.

3. Verfahren nach einem oder mehreren der Ansprüche 1 und 2, dadurch gekennzeichnet, daß die Haftklebefolie mit einer abgedunkelten bzw. lichtundurchlässigen Platte beschwert wird.

4. Verfahren nach einem oder mehreren der Ansprüche 1 bis 3, dadurch gekennzeichnet, daß die mit den Milben beladene Haftklebefolie (zum Zweck des störungsfreien Abzählens und Mikroskopierens) auf ihrer Klebeseite mit einer durchsichtigen, nichtklebenden Schutzfolie überschichtet wird.

5. Verfahren nach einem oder mehreren der Ansprüche 1 bis 4, dadurch gekennzeichnet, daß man eine Haftklebefolie einsetzt, deren Klebeschicht Stoffe enthält, die die Milben zusätzlich anlocken.

6. Verfahren nach einem oder mehreren der Ansprüche 1 bis 5, dadurch gekennzeichnet, daß man eine Haftklebefolie einsetzt, deren Klebeschicht Stoffe enthält, die zu einer vereinfachten Erkennung der Milben führen.

7. Verfahren nach einem oder mehreren der Ansprüche 1 bis 6, dadurch gekennzeichnet, daß man eine Haftklebefolie einsetzt, deren Klebeschicht Stoffe enthält, die zusammen mit einem Körperbestandteil der Milben einen Farbstoff entwickeln, z.B. einen Azofarbstoff mit Hilfe einer Kupplungskomponente und einem Eiweißabbauprodukt, wie z.B. Guanin aus dem Ausscheidungstrakt der betreffenden Milben.

8. Verfahren nach einem oder mehreren der Ansprüche 4 bis 7, dadurch gekennzeichnet daß man die Schutzfolie mit einer Reaktionskomponente ausrüstet, die der vereinfachten Erkennung der Milben dient, z.B. eine Azokupplungskomponente oder ein geeignetes Extraktionsmittel.

9. Verfahren nach einem oder mehreren der vorstehenden Ansprüche, dadurch gekennzeichnet, daß man den zu prüfenden Gegenstand auf seiner Rückseite der Einwirkung von Wärme unterwirft und die Milben zu einer gesteigerten Wanderung an die Vorderseite bzw. Oberfläche veranlaßt.

10. Verfahren nach Anspruch 9, dadurch gekennzeichnet, daß man die Wärmeeinwirkung mittels einer flächenförmigen Wärmequelle durchführt.

11. Anwendung des Verfahrens gemäß den vorstehenden Patentansprüchen für die Bekämpfung (Entfernung) noch lebender Hausstaubmilben in einem textilen, flächenförmigen Gegenstand, wobei man den (gegen lebenden Milbenbefall) zu bekämpfenden Gegenstand auf seiner Rückseite der Einwirkung von Wärme unterwirft, dabei die Milben zu einer gesteigerten Wanderung an die Vorderseite bzw. Oberfläche des Gegenstands veranlaßt und die ausgewanderten Milben dann aufnimmt sowie gegebenenfalls identifiziert.

## Claims

1. A method of detecting living house dust mites in a flat textile article, characterised in that the mites in the article which is to be examined (for infestation with living mites) are allowed to migrate to the front or surface of the article, the mites which have migrated out are picked up there by means of an adhesive film and are then, if appropriate, counted, evaluated in terms of their distribution and identified microscopically.

2. A method according to claim 1, characterised in that an adhesive film is used which, while picking up the mites, does not release any adhesive onto the article which is to be examined.

3. A method according to one or more of claims 1 and 2, characterised in that the adhesive film is

12

weighted with a darkened or opaque plate.

4. A method according to one or more of claims 1 to 3, characterised in that the adhesive film bearing the mites (for the purpose of trouble-free counting and microscopy) is covered, on its adhesive side, with a transparent, non-adhering protective film.

5. A method according to one or more of claims 1 to 4, characterised in that an adhesive film is used the adhesive layer of which contains substances which additionally attract the mites.

6. A method according to one or more of claims 1 to 5, characterised in that an adhesive film is used the adhesive layer of which contains substances which lead to easier recognition of the mites.

7. A method according to one or more of claims 1 to 6, characterised in that an adhesive film is used the adhesive layer of which contains substances which together with a constituent of the bodies of the mites develop a dye, e.g. an azo dye, by means of a coupling component and a protein breakdown product, such as guanine, from the excretory tract of the mites in question.

8. A method according to one or more of claims 4 to 7, characterised in that the protective film is provided with a reactant which serves to facilitate recognition of the mites, e.g. an azo coupling component or a suitable extraction agent.

9. A method according to one or more of the preceding claims, characterised in that the article to be examined is exposed to the action of heat on its reverse side and the mites are encouraged to migrate more to the front or top surface.

10. A method according to claim 9, characterised in that the heating effect is applied by means of a flat heat source.

11. Use of the method according to the preceding claims for combating (eliminating) living house dust mites from a flat textile article, in which the article which is to be treated (to combat living mite infestation) is subjected to the action of heat on its reverse side, the mites are thereby encouraged to migrate more to the front or top surface of the article and the mites which have migrated out are then picked up and, if appropriate, identified.

**Revendications**

1. Procédé pour la détermination des mites de poussière domestique encore vivantes dans une matière ou objet de forme plate, textile, caractérisé en ce que, sur la matière ou objet à examiner (en ce qui concerne l'infestation par des mites vivantes), on laisse migrer les mites vers la face ou respectivement la surface de la matière ou objet, on y prélève les mites ayant migré au moyen d'une feuille auto-adhésive et on les dénombre éventuellement ensuite, on estime leur répartition et on les identifie microscopiquement.

2. Procédé selon la revendication 1, caractérisé en ce qu'on utilise une feuille auto-adhésive qui prélève certes les mites, mais ne cède par d'adhésif à la matière ou objet à examiner.

3. Procédé selon l'une ou plusieurs des revendications 1 et 2, caractérisé en ce que la feuille auto-adhésive est chargée d'une plaque obscurcie ou respectivement opaque.

4. Procédé selon l'une ou plusieurs des revendications 1 à 3, caractérisé en ce que la feuille auto-adhésive chargée avec les mites (dans le but du dénombrement et de l'examen microscopique sans pertubation) est recouverte de son côté collant d'une feuille protectrice non-collante, transparente.

5. Procédé selon l'une ou plusieurs des revendications 1 à 4, caractérisé en ce qu'on utilise une feuille auto-adhésive dont la couche d'adhésif contient des matières qui de plus attirent les mites.

6. Procédé selon l'une ou plusieurs des revendications 1 à 5, caractérisé en ce qu'on utilise une feuille auto-adhésive dont la couche d'adhésif contient des matières qui conduisent à une reconnaissance simplifiée des mites.

7.   Procédé selon l'une ou plusieurs des revendications 1 à 6, caractérisé en ce qu'on utilise une feuille auto-adhésive dont la couche d'adhésif contient des matières qui, ensemble avec un constituant du corps des mites, développent un colorant par exemple un colorant azo à l'aide d'un composant de couplage et d'un produit de protéolyse, comme par exemple la guanine provenant du tractus d'excrétion des mites correspondantes.

8.   Procédé selon l'une ou plusieurs des revendications 4 à 7, caractérisé en ce qu'on on munit la feuille protectrice d'un composant de réaction qui sert à la reconnaissance simplifiée des mites, par exemple un composant de couplage azo ou un agent d'extraction approprié.

9.   Procédé selon l'une ou plusieurs des revendications précédentes, caractérisé en ce qu'on soumet la matière ou objet à examiner à l'effet de la chaleur sur sa partie arrière et en ce que l'on provoque une migration augmentée des mites sur la face ou respectivement la surface.

10.  Procédé selon la revendication 9, caractérisé en ce qu'on effectue l'application de la chaleur au moyen d'une source de chaleur de forme plate.

11.  Utilisation du procédé selon les revendications précédentes pour lutter (éliminer) les mites de poussière domestique encore vivantes dans une matière ou objet de forme plate, textile, selon laquelle on soumet la matière ou objet à traiter (contre une infestation par des mites vivantes) à l'effet de la chaleur sur sa face arrière, provoquant ainsi une migration augmentée des mites vers la face (avant) ou respectivement surface de la matière ou objet et oh prélève ensuite les mites ayant migré ainsi qu'éventuellement on les identifie.

Abbildung

Ausschnitt